# EUROPÄISCHE PATENTSCHRIFT

(11) **EP 1 017 669 B1**
(45) Veröffentlichungstag und Bekanntmachung des Hinweises auf die Patenterteilung: **03.04.2002**
(21) Anmeldenummer: 98947530.6
(22) Anmeldetag: 08.09.1998
(51) Int. Cl.: C07C 253/30, C07C 255/24, B01J 23/745

(54) **VERFAHREN ZUR HERSTELLUNG VON ALIPHATISCHEN ALPHA, OMEGA-AMINONITRILEN**
METHOD FOR PRODUCING ALIPHATIC ALPHA, OMEGA-AMINO NITRILES
PROCEDE DE PRODUCTION D'ALPHA, OMEGA-AMINONITRILES ALIPHATIQUES

(30) Priorität: 24.09.1997 DE 19742221
(43) Veröffentlichungstag der Anmeldung: 12.07.2000
(73) Patentinhaber: BASF AKTIENGESELLSCHAFT, 67056 Ludwigshafen (DE)
(72) Erfinder: VOIT, Guido, D-67251 Freinsheim (DE); ANSMANN, Andreas, D-69168 Wiesloch (DE); FISCHER, Rolf, Hartmuth, D-69121 Heidelberg (DE); BASSLER, Peter, D-68519 Viernheim (DE); LUYKEN, Hermann, D-67069 Ludwigshafen (DE); MERGER, Martin, D-67227 Frankenthal (DE); FLICK, Klemens, D-76863 Herxheim (DE)
(86) Internationale Anmeldenummer: EP9805685
(87) Internationale Veröffentlichungsnummer: WO9915497

(56) Entgegenhaltungen:
- EP-A- 0 223 439
- WO-A-98/11059
- DE-A- 2 429 293
- DE-A- 4 446 893
- DE-A- 19 636 765

## Beschreibung

Die vorliegende Erfindung betrifft ein Verfahren zur Herstellung von aliphatischen alpha, omega-Aminonitrilen in Gegenwart eines Katalysators, sowie Katalysatoren, die für die Hydrierung geeignet sind.

Aus DE-A 44 468 93 und WO-A-9 620 166 ist ein Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart eines Lösungsmittels und eines Katalysators bekannt, indem man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und.
(b) von 0,01 bis 25, vorzugsweise von 0,1 bis 5 Gew.-%, bezogen auf (a), eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a), einer Verbindung auf der Basis eines Alkalimetalles oder eines Erdalkalimetalles, enthält,
mit der Maßgabe, daß die Komponente (a) nicht auf der Basis von Eisen oder Eisen und einem der Metalle, ausgewählt aus der Gruppe, bestehend aus Cobalt, Ruthenium und Rhodium, besteht, wenn (b) ein Promotor auf der Basis eines Metalles, ausgewählt aus der Gruppe, bestehend aus Titan, Mangan, Chrom und Molybdän, ist sowie mit der weiteren Maßgabe, daß, wenn als Komponente (a) eine Verbindung auf der Basis von nur Ruthenium oder Rhodium oder .Ruthenium und Rhodium oder Nickel und Rhodium gewählt wird, der Promotor- (b) .gewünschtenfalls entfallen kann.

Nachteilig bei diesem Verfahren ist die Bildung von Nebenprodukten, die sich nur sehr schwer von den alpha,omega-Aminonitrilen, wie 6-Aminocapronitril oder gegebenenfalls weiteren Wertprodukten, wie Adipodinitril und Hexamethylendiamin im Falle von 6-Aminocapronitril als Alpha, omega - Aminonitril, abtrennen lassen.

So bilden sich beispielsweise im Falle der Hydrierung von Adipodinitril zu 6-Aminocapronitril und Hexamethylendiamin in wechselnden Mengen unter anderem 1-Amino-2-cyanocyclopenten (ICCP), 2-Aminomethylcyclopentylamin (AMCPA), 1,2-Diaminocyclohexan (DCH) und Bishexamethylentriamin (BHMTA). Aus der US-A 3 696 153 ist bekannt, daß sich AMCPA und DCH nur sehr schwer von Hexamethylendiamin abtrennen lassen.

Ferner ist die Standzeit der Katalysatoren bei diesem Verfahren nicht voll befriedigend.

Die ältere DE-Patentanmeldung 196 36 765.4 (veröffentlicht am 12/3/98) beschreibt ein ähnliches Verfahren wie die vorliegende Anmeldung, jedoch ist bei dem in 196 36 765.4 beschriebenen Verfahren die 6-Aminocapronitril (ACN)-Selektivität von dem Alter (Standzeit) der verwendeten Katalysatoren abhängig, es sei denn, man trennt den Phosphor im eingesetzten Adipodinitril (ADN) vorher ab. Die Phosphor-Abtrennung ist aber technisch aufwendiger und es ist wünschenswert diesen Schritt zu umgehen.

Aufgabe der vorliegenden Erfindung war es daher, ein Verfahren zur Herstellung von aliphatischen alpha, omega-Aminonitrilen durch partielle Hydrierung von aliphatischen alpha, omega-Dinitrilen in Gegenwart eines Katalysators zur Verfügung zu stellen, das die genannten Nachteile nicht aufweist und das die Herstellung von alpha, omega-Aminonitrilen mit hoher Selektivität auf technisch einfache und wirtschaftliche Weise ermöglicht, hohe Standzeiten bei praktisch unverändertem Umsatz und nach wie vor hoher alpha, omega-Aminonitrilselektivität hat.

Demgemäß wurde ein Verfahren zur Herstellung von aliphatischen alpha, omega-Aminonitrilen durch partielle Hydrierung von aliphatischen alpha, omega-Dinitrilen in Gegenwart eines Katalysators, der
(a) Eisen oder eine Verbindung auf der Basis von Eisen oder deren Gemische enthält, dadurch gekennzeichnet, daß der Katalysator
(b) von 0,01 bis 5 Gew.-% bezogen auf (a) eines Promotors auf der Basis von 2,3,4 oder 5 Elementen ausgewählt aus der Gruppe bestehend aus Aluminium, Silizium, Zirkonium, Titan und Vanadium sowie
(c) von 0 bis 0,5 Gew.-% bezogen auf (a) einer Verbindung auf der Basis eines Alkali- oder Erdalkalimetalls, enthält, und daß das verwendete alpha, omega-Dinitril 1,0 Gew.-ppm oder mehr Phosphor enthält,
gefunden.

Weiterhin wurden Katalysatoren, die durch die Reduktion und gegebenenfalls anschließende Passivierung eines Magnetits erhältlich sind, enthaltend
(a) Eisen oder eine Verbindung auf der Basis von Eisen oder deren Gemische,
(b) 0,01 bis 5 Gew.-% bezogen auf (a) eines Promotors auf der Basis von 2, 3, 4, oder 5 Elementen ausgewählt aus der Gruppe bestehend aus Aluminium, Silizium, Vanadium, Titan und Zirkonium,
(c) 0 bis 0,5 Gew.-% bezogen auf (a) einer Verbindung auf der Basis eines Alkalimetalls oder Erdalkalimetalls,
wobei die Katalysatoren eine BET-Oberfläche von 3 bis 10 m²/g, ein Gesamtporenvolumen von 0,05 bis 0,2 ml/g, einen mittleren Porendurchmesser von 0,03 bis 0,1 µm und einen Porenvolumenanteil im Bereich von 0,01 bis 0,1 µm von 50 bis 70 % haben, gefunden.

Bevorzugte Katalysator-Vorläufer sind solche, in denen die Komponente (a) zu 90 bis 100 Gew.-%, bevorzugt 92 bis 99 Gew.-%, bezogen auf (a) Eisenoxide, Eisenhydroxide, Eisenoxyhydroxide oder deren Gemische enthält. Als solche kommen beispielsweise Eisen-(III)-oxid, Eisen- (II, III)-oxid, Eisen-(II)-oxid, Eisen-(II)-hydroxid, Eisen-(III)-hydroxid oder Eisenoxydhydroxid wie FeOOH, in Betracht. Verwendet werden können synthetisch hergestellte oder natürlich vorkommende Eisenoxide, Eisenhydroxide oder Eisenoxyhydroxide, wie Magneteisenstein (Magnetit), der im Idealfall mit Fe₃O₄ beschrieben werden kann, Brauneisenstein, der im Idealfall mit Fe₂O₃ · H₂O beschrieben werden kann, oder Roteisenstein (Hämatit), der im Idealfall mit Fe₂O₃ beschrieben werden kann.

Bevorzugte Katalysator-Vorläufer sind-weiterhin solche, in denen Komponente (b) von 0,01 bis 5 Gew.-%, vorzugsweise 0,1 bis 4 Gew.-%, insbesondere 0,1 bis 2 Gew.-% eines Promotors auf der Basis von 2, 3, 4 oder 5 Elementen ausgewählt aus der Gruppe bestehend aus Aluminium, Zirkonium, Silizium, Titan und Vanadium enthalt.

Bevorzugte Katalysator-Vorläufer sind weiterhin solche, in denen Komponente (c) von 0 bis 0,5 Gew.-%, vorzugsweise 0,02 bis 0,2 Gew.-%, einer Verbindung auf der Basis eines Alkali- oder Erdalkalimetalls, vorzugsweise ausgewählt aus der Gruppe bestehend aus Lithium, Natrium, Kalium, Rubidium, Cäsium, Magnesium und Calcium enthält.

Bei den erfindungsgemäßen Katalysatoren kann es sich um Voll- oder Trägerkatalysatoren handeln. Als Trägermaterialien kommen beispielsweise poröse Oxide wie Aluminiumoxid, Siliziumdioxid, Alumosilikate, Lanthanoxid, Titandioxid, Zirkondioxid, Magnesiumoxid, Zinkoxid, und Zeolithe sowie Aktivkohle oder Mischungen davon in Betracht.

Die Herstellung erfolgt in der Regel derart, daß man Vorläufer der Komponente (a) gewünschtenfalls zusammen mit Vorläufern der Promotoren Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), gewünschtenfalls (b) und (c) auf den Träger nach an sich bekannten Methoden aufsprüht.

Als Vorläufer der Komponenten (a) kommen in der Regel gut wasserlösliche Salze des Eisens wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle und Halbmetalle wie Nitrate, Chloride, Acetate, Formiate und Sulfate in Betracht, vorzugsweise Nitrate.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 200 bis 500, vorzugsweise von 250 bis 400°C 2 bis 24 h einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 l pro 1 Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die BET-Oberfläche der erfindungsgemäßen Katalysatoren, bestimmt durch N₂-Adsorption nach DIN 66131, liegt im Bereich von 3 bis 10 m²/g.

Das Gesamtporenvolumen der erfindungsgemäßen Katalysatoren, bestimmt mit der Hg-Porosimetrie nach DIN 66133, liegt im Bereich von 0,05 bis 0,2 ml/g.

Der mittlere Porendurchmesser der erfindungsgemäßen Katalysatoren, errechnet aus der Porenvolumenverteilung, die mit der Hg-Porosimetrie nach DIN 66133 bestimmt wurde, liegt im Bereich von 0,03 bis 0,1 µm.

Der Porenvolumenanteil der Poren mit einer Dimension von 0,01 bis 0,1 µm der erfindungsgemäßen Katalysatoren, abgelesen aus der Porenvolumenverteilung, die mit der Hg-Porosimetrie nach DIN 66133 bestimmt wurde, beträgt 50 bis 70 Vol.-%.

Die Katalysatoren können als Festbettkatalysatoren in Sumpf- oder Rieselfahrweise oder als Suspensionskatalysatoren eingesetzt werden.

Als Ausgangsstoffe im erfindungsgemäßen Verfahren werden aliphatische alpha, omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Die Dinitrile der allgemeinen Formel I enthalten 1,0 Gew.-ppm oder mehr Phosphor. Gut geeignet sind Dinitrile, deren Phosphor-gehalt, bestimmt mit Atomemissionsspektroskopie nach saurem Aufschluß des ADN, im Bereich von 1 bis 100 Gew.-ppm, vorzugsweise im Bereich von 1 bis 20 Gew.-ppm, bezogen auf das Dinitril I oder das Dinitrilgemisch, enthaltend Dinitrile der allgemeinen Formel I, liegt. Der Phosphor kann in mannigfaltiger Form im Gemisch mit dem Dinitril I oder den Dinitrilen I vorliegen, so zum Beispiel als organisches Phosphit oder Phosphin oder deren jeweiligen Zersetzungs- oder Folgeprodukte. Als sehr gut geeignet haben sich Dinitrile I erwiesen, die aus alpha, omega-Dienen mit Cyanwasserstoff - wie beispielsweise in Weissermel, Arpe, Industrielle Organische Chemie, 2. Auflage, Seiten 233 bis 234 (1978), beschrieben - hergestellt wurden. Besonders gut geeignet ist alpha, omega-Adipodinitril, das durch Cyanwasserstoffaddition an 1,3-Butadien, beispielsweise wie in Weissermel, Arpe, Industrielle Organische Chemie, 2. Auflage, Seiten 233 bis 234 (1978), beschrieben, erhältlich ist.

Nach dem erfindungsgemäßen Verfahren werden die vorstehend beschriebenen Dinitrile I vorzugsweise in Gegenwart eines Lösungsmittels unter Verwendung eines Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 30, vorzugsweise von 3 bis 30, besonders bevorzugt von 4 bis 9 MPa. Die Verweilzeiten sind im wesentlichen von der gewünschten Ausbeute, Selektivität, und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 80 bis 120°C und einen Druck in der Regel im Bereich von 2 bis 40, vorzugsweise von 3 bis 30 MPa wählt. Bevorzugt führt man die pärtielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Die partielle Hydrierung kann man in einem üblichen, hierfür geeigneten Reaktor durchführen.

Bei der Hydrierung erhält man eine Mischung, die 6-Aminocapronitril, Hexamethylendiamin und Adipodinitril enthält.

Die Abtrennung von 6-Aminocapronitril, Hexamethylendiamin und einem im wesentlichen Adiponitril enthaltenden Teil von der Mischung kann in an sich bekannter Weise, vorzugsweise destillativ, beispielsweise gemäß der DE-A 195 002 22 oder der Deutschen Anmeldung 19 548 289.1, gleichzeitig oder nacheinander erfolgen.

Das nach dem erfindungsgemäßen Verfahren erhaltene Adipodinitril kann erneut für die partielle Hydrierung zu Hexamethylendiamin und 6-Aminocapronitril verwendet werden, wobei durch saure Behandlung -des rückzuführenden ACN eine Aufpegelung von Nebenprodukten vermieden wird, die eine spezifikationsgerechte Herstellung von Hexamethylendiamin und/oder 6-Aminocapronitril verhindern und/oder die Standzeit des Katalysators für die partielle Hydrierung negativ beeinflussen.

Nach dem erfindungsgemäßen Verfahren erhält man alpha, omega-Aminonitrile in guten Selektivitäten. Des weiteren weisen die erfindungsgemäß eingesetzten Katalysatoren ein deutlich längere Standzeit, bei gleichzeitig hoher ACN-Selektivität auf, als vergleichbare Katalysatoren aus dem Stand der Technik. Die alpha, omega-Aminonitrile sind wichtige Ausgangsverbindungen zur Herstellung von cyclischen Lactamen, insbesondere 6-Aminocapronitril für Caprolactam.

In den Beispielen bedeuten:
- ADN =: Adipodinitril
- ACN =: 6-Aminocapronitril
- HMD =: Hexamethylendiamin
- DCH =: cis + trans-1,2-Diaminocyclohexan
- AMCPA =: 1-Amino-2-aminomethylcyclopentan

### Beispiel 1

### a) Katalysatorherstellung

Der Katalysator wurde hergestellt durch sechsstündiges Tempern eines Magnetiterzes bei 150 °C unter Stickstoff. Das verwendete Magnetiterz hatte folgende Zusammensetzung: 72 Gew.-% Fe, 0,07 Gew.-% Al, 0,03 Gew.-% Ca, 0,04 Gew.-% Mg, 0,11 Gew.-% Si, 0,01 Gew.-% Ti, Rest Sauerstoff. Die Summe der Promotoren aus Gruppe b) liegt bei 0,19 Gew.-%, die. Summe der Promotioren aus Gruppe c), gerechnet als Oxide, liegt bei 0,11 Gew.-%.

Der abgekühlte Schmelzblock wurde im Backenbrecher zerkleinert und eine Siebfraktion der Teilchengröße 3 bis 6 mm ausgesiebt. Der oxidische Katalysator wurde im H₂/N₂-Strom bei 450°C 72 Stunden lang reduziert. Nach Abkühlen unter Stickstoff auf Raumtemperatur wurde der Fe-Kätalysator mit einem N₂/Luft-Strom passiviert (24 h mit 1 % Luft in Stickstoff), wobei darauf geachtet wurde, daß die Temperatur im Katalysatorbett nicht über 45°C anstieg.

### b) Partielle Hydrierung von ADN zu ACN

Ein Rohrreaktor (Länge 180 cm, d = 30 mm) wurde mit 740 ml (1819 g) der nach (a) hergestellten Katalysatormasse befüllt und drucklos im Wasserstoffstrom (500 Nl/h) reduziert. Dabei wurde die Temperatur innerhalb von 24 h von 30°C auf 340°C angehoben und anschließend 72 h bei 340°C gehalten.

Nach Absenken der Temperatur wurde dem Reaktor bei 250 bar ein Gemisch aus 400 ml/h ADN, welches aus 1,3-Butadien und Cyanwasserstoff (HCN) hergestellt wurde und einen Phosphor-Gehalt von 4 Gew.-ppm hatte, 660 ml/h Ammoniak und 500 Nl/h Wasserstoff zugeführt.

Nach einer. Laufzeit von 2000 h bei einer Reaktionstemperatur von-120 °C war, bei über die gesamte Laufzeit konstantem Umsatz und konstanter Gesamtselektivität (ACN + HMD) von 99 %, die ACN-Selektivität nur unwesentlich von 50 % auf 48 % abgefallen.

Der Gehalt an DCH im Hydrieraustrag betrug 2000 Gew.-ppm, bezogen auf HMD.

Der Gehalt an AMCPA im Hydrieraustrag betrug 5.0 Gew.-ppm, bezogen auf HMD.

### Vergleichsbeispiel

### a) Katalysatorherstellung

Durch Tempern eines Gemischs aus Magnetit, Kaliumcarbonat, Al₂O₃, Calciumcarbonat, Brechen der erstarrten Schmelze und Sieben gemäß A.B. Stiles, T.A. Koch, Catalyst Manufacture (1995) S. 167/68 wurde eine oxidische Masse folgender Zusammensetzung erhalten:
1,1 Gew.-% K₂O, 3,0 Gew.-% Al₂O₃, 2,3 Gew.-% CaO, 0,11 Gew.-% Si, 0,01 Gew.-% Ti, Rest Fe-Oxide.

Anschließend wurde diese Masse im N₂/H₂-Strom bei 450°C 72 h reduziert, bei Raumtemperatur mit einem N₂/Luft-Gemisch (24 h mit 1 % Luft in Stickstoff), wobei die Temperatur im Katalysatorbett nicht über 45°C stieg, passiviert und mit Wasser 7 Tage gewaschen.

Die erhaltene Katalysatormasse hatte folgende Zusammensetzung:
1,2 Gew.-% Al, 0,74 Gew.-% Ca, 0,02 Gew.-% K, 0,11 Gew.-% Si, 0,01 Gew.-% Ti, Rest Fe/Fe-Oxid. Die Summe der Promotoren aus Gruppe b) liegt bei 1,32 Gew.-%, die Summe der Promotoren aus Gruppe c) gerechnet als Oxide, liegt bei 1,06 Gew.-%.

### b) Partielle Hydrierung von ADN zu ACN

Ein Rohrreaktor (Länge 180 cm, d = 30 mm) wurde mit 740 ml (1819 g) der nach a) hergestellten Katalysatormasse befüllt und drucklos im Wasserstoffstrom (500 Nl/h) reduziert. Dabei wurde die Temperatur innerhalb von 24 h von 30°C auf 340°C angehoben und anschließend 72 h bei 340°C gehalten.

Nach Absenken der Temperatur wurde dem Reaktor bei 250 bar ein Gemisch aus 400 ml/h ADN, welches aus 1,3-Butadien und Cyanwasserstoff (HCN) hergestellt wurde und einen Phosphor-Gehalt von 4 Gew.-ppm hatte, 660 ml/h Ammoniak und 500 Nl/h Wasserstoff zugeführt.

Nach einer Laufzeit von 2000 h bei einer Reaktionstemperatur von 120 °C war, bei über die gesamte Laufzeit konstantem Umsatz und konstanter Gesamtselektivität (ACN + HMD) von 99 %, die ACN-Selektivität von 40 % auf 25 % abgefallen.

Der Gehalt an DCH im Hydrieraustrag betrug 4000 Gew.-ppm, bezogen auf HMD.

Der Gehalt an AMCPA im Hydrieraustrag betrug 150 Gew.-ppm, bezogen auf HMD.

## Patentansprüche

1. Verfahren zur Herstellung von aliphatischen alpha,omega-Aminonitrilen durch partielle Hydrierung von aliphatischen alpha,omega-Dinitrilen in Gegenwart eines Katalysators, der
(a) Eisen oder eine Verbindung auf der Basis von Eisen oder deren Gemische enthält, **dadurch gekennzeichnet, daß** der Katalysator
(b) von 0,01 bis 5 Gew.-% bezogen auf (a) eines Promotors auf der Basis von 2,3,4 oder 5 Elementen ausgewählt aus der Gruppe bestehend aus Aluminium, Silizium, Zirkonium, Titan und Vanadium sowie
(c) von 0 bis 0,5 Gew.-% bezogen auf (a) einer Verbindung auf der Basis eines Alkali- oder Erdalkalimetalls, enthält, und daß das verwendete aliphatische alpha, omega-Dinitril 1,0 Gew.-ppm oder mehr Phosphor enthält.

2. Verfahren nach Anspruch 1, wobei der Katalysator durch Reduktion und gegebenenfalls anschließende Passivierung eines Magnetits erhältlich ist und eine BET-Oberfläche von 3 bis 10 m²/g, ein Gesamtporenvolumen von 0,05 bis 0,2 ml/g, einen mittleren Porendurchmesser von 0,03 bis 0,1 µm und einen Porenvolumenanteil im Bereich von 0,01 bis 0,1 µm von 50 bis 70 % hat.

3. verfahren nach den Ansprüchen 1 bis 2, wobei man als Verbindung auf der Basis von Eisen ein Eisenoxid oder Gemische von Eisenoxiden einsetzt.

4. Verfahren nach den Ansprüchen 1 bis 3, wobei man einem Promotor auf der Basis Aluminium, Silizium und Vanadium einsetzt.

5. Verfahren nach den Ansprüchen 1 bis 4, wobei der Katalysator ein Trägerkatalysator ist.

6. Verfahren nach den Ansprüchen 1 bis 5, wobei der Katalysator ein Vollkatalysator ist.

7. Verfahren nach den Ansprüchen 1 bis 6, wobei man als Dinitril Adipodinitril einsetzt, unter Erhalt von 6-Aminocapronitril.

8. Verfahren nach den Ansprüchen 1 bis 6 zur gleichzeitigen Herstellung von 6-Aminocapronitril und Hexamethylendiamin ausgehend von Adipodinitril durch
(1) partielle Hydrierung von Adipodinitril in Gegenwart eines Katalysators unter Erhalt einer Mischung enthaltend 6-Aminocapronitril, Hexamethylendiamin und Adipodinitril und
(2) Abtrennung von 6-Aminocapronitril und Hexamethylendiamin aus der Mischung.

9. Verfahren nach den Ansprüchen 1 bis 8, **dadurch gekennzeichnet, daß** man die Hydrierung in einer Suspension vornimmt.

10. Verfahren nach den Ansprüchen 1 bis 8, **dadurch gekennzeichnet, daß** man die Hydrierung in einem Festbettreaktor vornimmt.

11. Verfahren nach den Ansprüchen 1 bis 10, **dadurch gekennzeichnet, daß** das eingesetzte alpha, omega-Dinitril durch Hydrocyanierung - in Gegenwart von Phosphor-haltigen Katalysatoren - eines um zwei Kohlenstoffatome ärmeren alpha, omega-Diens erhalten wurde.

## Claims

1. Process for the preparation of aliphatic alpha, omega-aminonitriles by partial hydrogenation of aliphatic alpha,omega-dinitriles in the presence of a catalyst which
(a) contains iron or a compound based on iron or mixtures thereof, wherein the catalyst
(b) contains from 0.01 to 5% by weight, based on (a), of a promoter based on 2, 3, 4 or 5 elements selected from the group consisting of aluminum, silicon, zirconium, titanium and vanadium and
(c) from 0 to 0.5% by weight, based on (a), of a compound based on an alkali metal or on an alkaline earth metal, and wherein the alpha,omega-dinitrile used contains 1.0 ppm by weight or more of phosphorus.

2. The process as claimed in claim 1, wherein the catalyst is obtainable by reduction and, if required, subsequent passivation of a magnetite and has a BET surface area of from 3 to 10 m²/g, a total pore volume of from 0.05 to 0.2 ml/g, an average pore diameter of from 0.03 to 0.1 µm and a pore volume fraction of from 50 to 70% in the range from 0.01 to 0.1 µm.

3. A process as claimed in claims 1 and 2, wherein an iron oxide or a mixture of iron oxides is used as the compound based on iron.

4. A process as claimed in any of claims 1 to 3, wherein a promoter based on aluminum, silicon and vanadium is used.

5. A process as claimed in claims 1 to 4, wherein the catalyst is a supported catalyst.

6. A process as claimed in any of claims 1 to 5, wherein the catalyst is an unsupported catalyst.

7. A process as claimed in any of claims 1 to 6, wherein the dinitrile used is adiponitrile, 6-aminocapronitrile being obtained.

8. A process as claimed in any of claims 1 to 6 for the simultaneous preparation of 6-aminocapronitrile and hexamethylenediamine starting from adiponitrile by
(1) partial hydrogenation of adiponitrile in the presence of a catalyst, a mixture containing 6-aminocapronitrile, hexamethylenediamine and adiponitrile being obtained, and
(2) isolation of 6-aminocapronitrile and hexamethylenediamine from the mixture.

9. A process as claimed in any of claims 1 to 8, wherein the hydrogenation is carried out in a suspension.

10. A process as claimed in any of claims 1 to 8, wherein the hydrogenation is carried out in a fixed-bed reactor.

11. A process as claimed in any of claims 1 to 10, wherein the alpha,omega-dinitrile used was obtained by hydrocyanation - in the presence of phosphorus-containing catalysts - of an alpha,omega-diene having two fewer carbon atoms.

## Revendications

1. Procédé de préparation d'alpha, oméga-aminonitriles aliphatiques par hydrogénation partielle d'alpha, oméga-dinitriles aliphatiques en présence d'un catalyseur,
(a) qui contient du fer ou un composé à base de fer ou leurs mélanges,
**caractérisé en ce que** le catalyseur contient
(b) de 0,01 à 5% en poids, par rapport à (a), d'un promoteur à base de 2, 3, 4 ou 5 éléments choisis parmi le groupe constitué de l'aluminium, du silicium, du zirconium, du titane et du vanadium, ainsi que
(c) de 0 à 0,5% en poids, par rapport à (a), d'un composé à base d'un métal alcalin ou alcalino-terreux, et **en ce que** l'alpha, oméga-dinitrile aliphatique utilisé contient 1,0 ppm en poids ou davantage de phosphore.

2. Procédé suivant la revendication 1, dans lequel le catalyseur peut être obtenu par réduction et éventuellement par passivation ultérieure d'une magnétite et possède une surface spécifique BET de 3 à 10 m²/g, un volume poreux global de 0,05 à 0,2 ml/g, un diamètre moyen des pores de 0,03 à 0,1 µm et une fraction volumique des pores dans la gamme de 0,01 à 0,1 µm de 50 à 70%.

3. Procédé suivant l'une des revendications 1 et 2, dans lequel on met en oeuvre, comme composé à base de fer, un oxyde de fer ou des mélanges d'oxydes de fer.

4. Procédé suivant l'une des revendications 1 à 3, dans lequel on met en oeuvre un promoteur à base d'aluminium, de silicium et de vanadium.

5. Procédé suivant l'une des revendications 1 à 4, dans lequel le catalyseur est un catalyseur sur support.

6. Procédé suivant l'une des revendications 1 à 5, dans lequel le catalyseur est un catalyseur massique.

7. Procédé suivant l'une des revendications 1 à 6, dans lequel on met en oeuvre, comme dinitrile, de l'adipodinitrile, avec obtention de 6-aminocapronitrile.

8. Procédé suivant l'une des revendications 1 à 6, pour la préparation simultanée de 6-aminocapronitrile et d'hexaméthylènediamine à partir d'adipodinitrile,
(1) par hydrogénation partielle d'adipodinitrile en présence d'un catalyseur avec obtention d'un mélange contenant du 6-aminocapronitrile, de l'hexaméthylènediamine et de l'adipodinitrile, et
(2) par isolement de 6-aminocapronitrile et d'hexaméthylènediamine à partir du mélange.

9. Procédé suivant l'une des revendications 1 à 8, **caractérisé en ce qu'**on effectue l'hydrogénation dans une suspension.

10. Procédé suivant l'une des revendications 1 à 8, **caractérisé en ce qu'**on effectue l'hydrogénation dans un réacteur à lit fixe.

11. Procédé suivant l'une des revendications 1 à 10, **caractérisé en ce que** l'alpha-oméga-dinitrile mis en oeuvre est obtenu par hydrocyanation, en présence de catalyseurs contenant du phosphore, d'un alpha-oméga-diène plus pauvre de deux atomes de carbone.
